# EUROPEAN PATENT APPLICATION

(11) **EP 1 797 834 A1**
(43) Date of publication of application: **20.06.2007**
(21) Application number: 06270098.4
(22) Date of filing: 08.12.2006
(51) Int. Cl.: A61B 17/15, A61B 17/17

(54) **Alignment guide**

(30) Priority: 16.12.2005 GB 0525637
(71) Applicant: Finsbury (Development) Limited, Randalls Road Leatherhead, Surrey KT22 7BA (GB)
(72) Inventor: Wozencroft, Robert Michael, Epsom, Surrey KT19 8SS (GB)
(74) Representative: Smaggasgale, Gillian Helen

(57) **Abstract**

An alignment guide for use in femoral head surgery comprising:
a support member (1);
a cannulated rod (3) supported by, and adjustable with respect to, the support member (1); and
a locator arm (4) having a proximal end connected to the support member and a distal end having location means (5) for location on a high point of the femoral head and a notch guard (9) which in use will extend around at least a part of the femoral neck.

## Description

The present invention relates to a tool for use in hip resurfacing operations. More particularly, it relates to an alignment guide for assisting in the correct machining of the femoral head such that a replacement femoral head can be correctly situated..

The efficient functioning of the hip joints is extremely important to the well being and mobility of the human body. Each hip joint is comprised by the upper portion of the upper leg bone (femur) which terminates in an offset bony neck surmounted by a ball-headed portion which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component can then be inserted into place. In some arrangements, the acetabular component may simply be held in place by a tight fit with the bone. However, in other arrangements, additional fixing means such as screws or bone cement may be used. The use of additional fixing means help to provide stability in the early stages after the prosthesis has been inserted. In some modem prosthesis, the acetabular component may be coated on its external surface with a bone growth promoting substance which will assist the bone to grow and thereby assist the holding of the acetabular component in place. The bone femoral head will be removed and the femur hollowed using reamers and rasps to accept the prosthesis. The stem portion will then be inserted into the femur.

In some cases, a femoral component of the kind described above may be replaced with components for use in femoral head resurfacing or for use in thrust plate technology.

Although the prosthesis being inserted when the head is being replaced or resurfaced or in thrust plate arrangements is relatively small, the requirement for the surgeon to obtain the necessary access to the hip joint means that it is necessary to make a large incision on one side of the hip. In one technique, a straight incision is made through the skin on the posterior edge of the greater trochanter. In some techniques this incision may be made when the hip is flexed to 45°. By known techniques, the muscles and tendons are parted and held by various retractors such that they do not interfere with the surgeons access to the hip joint. The hip is then dislocated to provide access to the head of the femur.

It will be acknowledged that it is essential that the replacement surface for the head of the femur should be precisely located in both angular and translation positions of the axis of the femoral neck of the implant. To assist this, in some techniques, the surgeon inserts a pin in the lateral femur. The desired position of the pin will be known from pre-operative analysis of the x-rays. The surgeon will measure the desired distance down the femur from the tip of the greater trochanter and the alignment pin is inserted through the vastus lateralis fibres. The alignment pin is inserted in a transverse direction into the mid-lateral cortex and directed upwardly towards the femoral head. The pin is left protruding so that an alignment guide can be hooked over the alignment pin. Suitable alignment guides include those known as the McMinn Alignment Guide available from Smith & Nephew Orthopaedics Limited.

These alignment guides of the kind described above generally comprise a hook or aperture which is placed over the alignment pin thus providing a good angular position for the axis of the implant in valgus, varus and ante-version of the neck. The guide will then be adjusted such that a cannulated rod is located such that the aperture therein is directed down the mid-lateral axis of the femoral neck. A stylus having been set to the desired femoral component size is positioned such that it can be passed around the femoral neck.

When the stylus can be passed around the femoral neck, the cannulated rod is locked in position. Once the guide is stabilised in this way fine adjustments can be made until the surgeon is happy that the guide is in the required position.

A guide wire can then be inserted through the cannulated rod. This guide wire is then used in the further surgery in which the femoral head is shaped to accept the prosthesis. This shaping involves removing the top of the head at an appropriate position and then machining the sides of the head using a sleeve cutter. These sleeve cutters are arranged such that the diameter cut will be correct for the replacement head size chosen and will bottom on the top of the cut head such that the teeth of the cutter do not dangerously over-sail the head-neck junction and cause soft tissue damage or neck notching.

Thus the machining procedure usually comprises the steps of drilling a well into the head of the femur, removing the drill, removing the top of the head of the femur, inserting a guide rod into the well, locating a sleeve cutter on the guide rod and cutting the head and optionally chamfer cutting the head. However, it will be understood that the order of the steps may be altered.

An alignment guide is generally used to ensure that the aperture drilled in the femoral head is both central to the femoral neck and at the correct angle of alignment to the femoral neck and that the shaping of the femoral head is accurate for the chosen head size.

It will therefore be understood that it is very important that the alignment guide is positioned correctly. Failure to do so may have the disastrous effect of allowing the machining of the cylinder of the head during the shaping procedure to "notch" into the neck of the femur. This will predispose the bone to early failure on load bearing.

Alternative improved alignment guides are described in European Patent publication nos 1588668 and 1588669 which are incorporated herein by reference. These improved alignment guides allow the required incision in the hip to be as small as possible and the amount of interaction with healthy tissue to be minimised. This is achievable as they do not require the alignment pin required by previous devices to be inserted. Where these guides are used, all of the surgical procedure takes place at the femoral head and the positioning and angling of the guide wire is taken, via the tools, from the femoral neck.

Other guides are known which are, in use, located on the femoral neck itself. These are used in a similar manner to those described above and may involve some adjustment by the surgeon to select the best position.

Whilst many of these alignment guides provide satisfactory results, there is a need for alternatives for situations where the surgeon is unable to use, or prefers not to use, the femoral neck as the basis used by the guide to assess the correct angle. It is therefore desirable to provide alternative guides which utilise the femoral head itself to provide the correct orientation for the alignment guide.

However, one problem associated with using the femoral head as the basis for correct position of the alignment guide is that the femoral head may have become misshapen due to the effects of, for example, arthritis and thus the orientation taken by the alignment guide may be incorrect which will lead to the angle at which the drilling for the guide wire occurs being not at the optimum orientation which in turn will mean that the machining of the head will not be correct and thus the placement of the head prosthesis may not be correct.

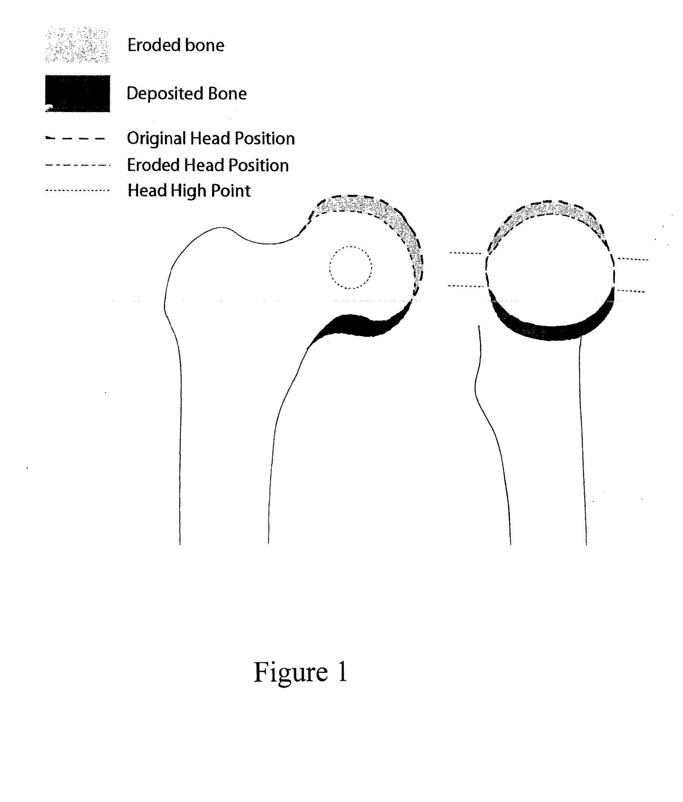

Figure 1 illustrates how the shape of the femoral head can alter due to the effects of arthritis. As illustrated, bone is eroded from the top surface of the femoral head. In addition, bone can be deposited on the bottom surface of the femoral head in the form of osteophytes. It will therefore be understood that a notional axis drawn between the high point on the top of the femoral head and the opposite high point on the bottom of the head would have as its centre a point which gradually moves downwardly through the head as the affects of the arthritic erosion and deposition increase.

For the purposes of this application, the references to the "top" and "bottom" surfaces of the femoral head are to the top and bottom of the femoral head when the leg is in the standing position. Similarly, any reference to the front and back of the femoral head will be those to the front and back of the body when the femoral head is located in the acetabular.

The front and back sides of the femoral head are not generally affected by the erosion and deposition of arthritis. Thus a notional axis passing through opposite high points on the front and back sides of the femoral head will pass through the centre of the head as it was prior to arthritic erosion and deposition occurring, i.e. the natural true center point of the femoral head. This centre point will be in line with the femoral neck. The high points on the front and back of the femoral head are also illustrated in Figure 1. It will therefore be understood that these high points provide a constant reference point. It has now been established that these high points may be used to accurately locate an alignment guide on the femoral head.

Thus according to the present invention there is provided an alignment guide for use in femoral head surgery comprising:
a support member;
a cannulated rod supported by, and adjustable with respect to, the support member; and
a locator arm having a proximal end connected to the support member and a distal end having location means for location on a high point of the femoral head and a notch guard which in use will extend around at least a part of the femoral neck.

The use of the alignment guide of the present invention enables the true centre of the femoral head centre to be correctly located. Utilising the femoral head high point as the point about which the alignment guide is orientated, means that any alteration in the shape or size of the femoral head due to, for example, arthritis, does not effect the correct alignment. The use of the high point of the femoral head has particular attractions since the natural femoral head has an anterior offset providing more flexion than if there was neutral offset.

The location means at the distal end of the locator arm may be of any suitable configuration. In one preferred arrangement, it will be of an annular configuration such that the apex of the high point will sit within the location means. It will be understood that the location means for interacting with the femoral head does not have to be of circular configuration nor be a complete circle provided that there is sufficient interaction around the high point of the head. It will be understood that whilst a circular configuration offers certain advantages, other configurations may be used.

The notch guard may be of any suitable configuration. In one arrangement, the notch guard may be a ring extending from the location means and generally angled thereto such that in use the ring will sit around the neck of the femur and serves as a notch guard. In femoral head resurfacing, cylindrical cutters are used to shape the sides of the head. These cutters are arranged such that the diameter of the cut will be correct to enable the replacement head size chosen to be fitted. These cutters will bottom on the top of the cut head such that the teeth of the cutter do not dangerously over-sail the head neck junction and cause soft tissue damage or neck notching. The notch guard provides a visual indication to the surgeon prior to starting cutting as to where the base of the cut will be for a particular head sized cutter. It will be understood that whilst the notch guard has been described as having a ring configuration any suitable arrangement may be used.

In one arrangement, the ring is a C-ring so that in use the guide can be placed in position by passing the neck of the femoral head through the open space in the ring. It will be understood that the size and position of the break in the C-ring may mean that the ring may be placed around the femoral neck in one orientation and then the device rotated to the desired position for surgery.

In another arrangement the ring may have a portion which is hinged to the remainder of the ring and which in an open orientation will enable the guide to be placed in position around the neck of the femoral head. The hinged portion can then be closed to complete the ring. Thus the hinged portion may be regarded as a gate or door.

In a still further arrangement the ring may be formed of two arms which are each hinged to allow movement to an open position such that the guide may be located in position. When in the closed position in use, the two arms will close around the neck of the femur. In one arrangement, the two arms will be sized such that when in the closed position, they complete a circle but they may simply form a segment of a circle. The two arms may be of the same or different lengths. Thus where the arms, when in the closed position, complete a circle, the two arms may meet at a point opposite the point of connection to the support arm or may be at another point on the circumference of the circle. In one alternative arrangement one arm may be fixed and the other may be adjustable thereto.

In a further alternative arrangement, the ring may be adjustable. For example, an iris which will expand to enable the ring to be passed over the femoral head and then contracted to fit around the neck of the femur.

In use, the notch guard will not normally be in contact with the femoral neck in use and does not play an active role in positioning the alignment guide. The notch guard will therefore be generally clear of any osteophytes located on the bottom of the femoral head such that there is no requirement to remove them.

In one arrangement, the alignment guide may include a support arm extending from the support member and connected thereto. The support arm will have a proximal end connected to the support member and a distal end which in use will be located on the femoral head on the opposite side to that contacted by the location means of the locator arm. The support arm may include contact means situated at the distal end thereof. Any suitable contact means may be used. In one arrangement, the contact means may be a locator means to locate the high point on the side of the femoral head contacted by the support arm. The locator means on the support arm, where present may be of the same or a different configuration to that located on the locator arm.

Where both the locator arm and the support arm are present at least one of the arms may be moveable from a first open position to a second clamping position. In this arrangement when in the first open position the alignment guide may be moved into position around the head of the femur and in the second clamping position can be clamped against the femoral head high point on the respective front and back face.

In one arrangement, both arms may be movable from the first open position to the second clamping position. However, in a preferred arrangement, one arm is fixed with reference to the support member and the other member is movable with respect thereto. In this arrangement, the movable arm may be connected to the support member and movable in relation thereto by any suitable means. In one arrangement the movable arm may be pivoted to the support member or to the fixed arm.

Where the movable arm is pivoted to the support member or the fixed arm, the movement of the movable arm about the pivot may simply be controlled by the operator. However, in a preferred arrangement, a means for causing the movement of the movable arm may be included. The means for causing movement may additionally act to lock the movable arm in position once the alignment guide is in position and the distal end of the arms are in position around the femoral head. In an alternative arrangement, a separate locking means may be used.

The movable arm may include a handle, which will generally be integral with the arm means and will extend on the proximal side of the pivot where present. The handle may include an aperture through which in use the surgeon may insert his thumb. The support member may include a handle which may include an aperture through which in use the surgeon may insert his fingers. Where both handles are present, it will be understood that the surgeon is provided with a convenient arrangement by which the alignment guide can be held with one hand. In an alternative arrangement, at least one aperture for insertion of the thumb and/or fingers may be provided on the support member.

The movable arm may preferably be movable by the operation of a screw means which passes through a threaded aperture in the movable arm. In the arrangement where the movable arm includes a handle, the threaded aperture through which the screw means passes may be in the handle portion i.e. at the proximal side of the pivot means. The screw means may act by simply pressing on the surface of the support means such that as the screw means is turned, the movable arm is caused to move relative to the fixed arm. However, one end of the screw means will generally be fixed by any suitable means to the support member or to the fixed arm. The screw means will generally include a means to facilitate turning of the screw means. The means to facilitate turning may be a knurled wheel.

The or each arm may be shaped along its length such that in use the distal ends will sit in the correct position on the femoral head.

As will be discussed in more detail below, at least one arm may include other features to enable the surgeon to verify the positioning of the alignment guide and to facilitate the various operation steps which have to be taken. Whilst these can be located on either of the arms, where two arms are present, and some may be located on one arm and some on the other, where present, for simplicity of manufacture and use, these additionally features will generally be provided on one arm which, in the arrangement in which one arm is fixed to the support member, will generally be located on the fixed arm such that the movable arm simply has the means for engaging with the high point of the femoral head. In this embodiment, in use the movable arm will generally be located on the side of the femoral head which has less visibility to the surgeon, normally the back side, and the fixed arm carrying the additional alignment features will be located on the side of the femoral head, usually the front, having the highest visibility.

The location means located at the distal end of the locator arm, which in the embodiment in which one arm is fixed will generally be the fixed arm, may include two indicator fingers extending in an arcuate arrangement to provide a visible guide to the surgeon as to the size of the replacement femoral head in relation to the natural femoral head and/or its positioning with the alignment guide in the location at which it has been placed.

As replacement femoral heads are available in a variety of sizes, a range of alignment guides may be provided wherein the locator arm on a particular size of guide, corresponds to a particular size of replacement head. However, a more cost effective arrangement is for a portion of the distal end of the locator arm to be demountable and interchangeable with other distal ends each having indicator arms representing different sized replacement heads.

The cannulated rod may be adjustable with respect to the support arrangement. In one arrangement the rod is a sliding fit in the support. Once in the desired position the cannulated rod will preferably be lockable such that once locked further movement is prevented. Any suitable locking means may be used with a locking screw being preferred.

The cannulated rod will in use enable the surgeon to position the guide wire. The cannulated rod may have a slot extending along at least a part of the length of the rod to assist in removing the tool from the guide wire once it is in position.

Teeth may be provided at the distal end thereof which can be driven into the surface of the femoral head. When driven into the head, these teeth help to clamp the alignment tool in position and further stabilise it.

The cannulated rod may additionally function as a measuring or gauging device and thus the surface of the rod may including measuring indicia to assist the surgeon to know how deep they have cut.

In femoral head resurfacing techniques, the surgeon will shape the head of the femur to fit within the cavity of the resurfacing prosthesis. This generally involves a number of shaping steps including the removal of the dome of the femoral head by means of a saw. It is important that the saw cut is made in the correct position so that an accurate positioning for the prosthesis can be achieved.

The position of the cut to remove the dome of the femoral head can be calculated from the top of the dome of the undamaged femoral head. However, to assist the surgeon a cutting guide may be located on the locator means. Where a locator arm is provided with interchangeable distal ends, the cutting guide may be located as part of the interchangeable portion so that the correct position of the cut for each size of replacement head is indicated simply by utilizing the correct distal end of arm for the size of head.

It is important for the correct operation of the hip prosthesis and the well-being of the patient that the prosthesis is correctly sited. As all of the machining of the femoral head is taken from the position of the guide wire inserted into the head, it is imperative that this is inserted as correctly as possible. The correct axis for insertion of the guide wire into the head of the femur is approximately 30 degrees from the sagittal plane axis of the femur and 20 degrees in anteversion to allow for natural offset in each position.

The alignment guide of the present invention preferably includes means to provide the surgeon with a visual indication of the varus/valgus angle. In one arrangement, this may simply be a varus flag extending from the alignment guide to enable the surgeon to visually confirm that the alignment guide is in the desired position.

However, in a preferred arrangement, a goniometer may be included. This may be integral with the alignment guide or in a preferred arrangement may be connectable to the alignment guide. In use the goniometer will point directly at the centre of the knee and provide the correct angle for the stem on the resurfacing head. The goniometer may be connectable to the alignment guide by any suitable means. In one arrangement, interlocking means may be provided on the distal end of one arm, preferably the locator arm, to engage corresponding features on the goniometer.

In order to indicate the correct anteversion angle, the alignment guide may additionally include antiversion indicating means. Whilst this may be integral with the guide and may be an antiversion flag extending from the alignment guide, in one preferred arrangement, it is separate therefrom and demountable from the alignment guide. In one arrangement, the antiversion indicating means may be connectable to the notch guard where present or to any other suitable position on the alignment guide. Where the antiversion indicating means is to be plugged onto the notch guard, the indicating means may simply clamp to the guard or the guard may be suitably shaped to have an interlocking means with a component of the indicating means. Suitable antiversion indicating means, include those having a Y shape fork the tines of which will sit along at least a portion of the notch guard where present and act to stabilise the alignment guide.

In an alternative arrangement, the antiversion indicating means could include a biting element such that the indicating means will connect with the femoral neck and also take a varus/valgus angle therefrom. The biting element may be of any suitable configuration. In one arrangement it may be a toothed block. The block may have a concave face between the teeth. The block may comprise four teeth, the teeth will preferably be configured and spaced on the block such that in use they interact with the inferior part of the neck of the femur to cause the tool to be angled at the optimum position. Thus the teeth will enable the tool to be clamped at the correct anteversion angle and at the correct angle from the sagittal plane with these angles being fixed by the femur itself. It is generally believed that there is a portion of the inferior femoral neck located from the head/neck junction of the femur to a position about 2 cms from the head/neck junction which is parallel to the optimum angle for the positioning of the stem of the prosthesis and hence this is often used as an alignment reference.

The optimum position of the tool may be achieved with four teeth in a generally square configuration. The teeth are preferably spaced at from about 10 to about 25 mm apart. They are most preferably spaced at about 15 mm.

According to a second aspect of the present invention there is provided a kit comprising at least one alignment guide in accordance with the above first aspect, a goniometer and an antiversion alignment guide.

Where the alignment guide has a demountable distal end to at least one arm the kit may include a plurality of distal ends representing various sizes of resurfacing heads.

The alignment guide of the present invention may be used in a method of preparing the head of a femur for femoral head resurfacing wherein the method comprises:
exposing the head of a femur;
locating the alignment guide according to the above first aspect on the head of the femur; and
machining the head of the femur.

To use the alignment guide most successfully an assessment of the head size should be made prior to machining and use of the guide in order to select the appropriate sized alignment guide or, where used, demountable locator means. The correctly selected guide or demountable locator means may provide a visual indication as to whether the correct size has been selected particularly in the embodiment including the fingers which indicate the size of the head. However, a more accurate method would be to measure the head size using, for example, calipers.

During the surgery, a well may be drilled into the head of the femur via the cannulated rod. This well may be the definite hole diameter required of approximately 8 mm and drilled to a depth determined by the tube touching the head. A check may be made with a stylus once the tool is removed and cylinder cutters used guided over a peg placed in the well. These cutters are arranged such that the diameter cut will be correct for the head size chosen and will bottom on the top of the cut head such that the teeth of the cutter do not dangerously over-sail the head-neck junction and cause soft tissue damage or neck notching.

Thus the method preferably comprises:
exposing the head of the femur;
locating the alignment guide according to the above first aspect on the head of the femur;
inserting a drill and drilling a well into the head of the femur;
removing the drill;
removing the alignment guide;
removing the top of the head of the femur;
inserting a guide rod into the well;
locating a sleeve cutter on the guide rod and cutting the head; and
optionally chamfer cutting the head.

The correct axis for insertion of the guide wire into the head of the femur is approximately 30 degrees from the sagittal plane axis of the femur and in anteversion to allow for the natural offset in each position. Thus the tool of the present invention is configured such that in use the cannulated bore will be located such that the guide wire or drill is inserted at the correct angle. The arrangement of the present invention allows the surgeon to place, and to visually check that the tool is in the correct orientation, and position centered on the femoral head-neck junction.

It will be understood that whilst the tool of the present invention offers particular advantages for minimal invasive surgery, it can also be used in conventional surgical techniques.

The tool of the present invention may be used with all sizes of resurfacing head.

The present invention will now be described by way of example with reference to the accompanying figures in which:
- Figure 1: is a schematic representation of a femoral head illustrating the femoral head position in a health femur and in a damaged femur;
- Figure 2: is a perspective view of the alignment guide of one embodiment of the present invention;
- Figure 3: is an exploded view of the alignment guide of the present invention illustrating the relationship of the components and including the antiversion indicator means;

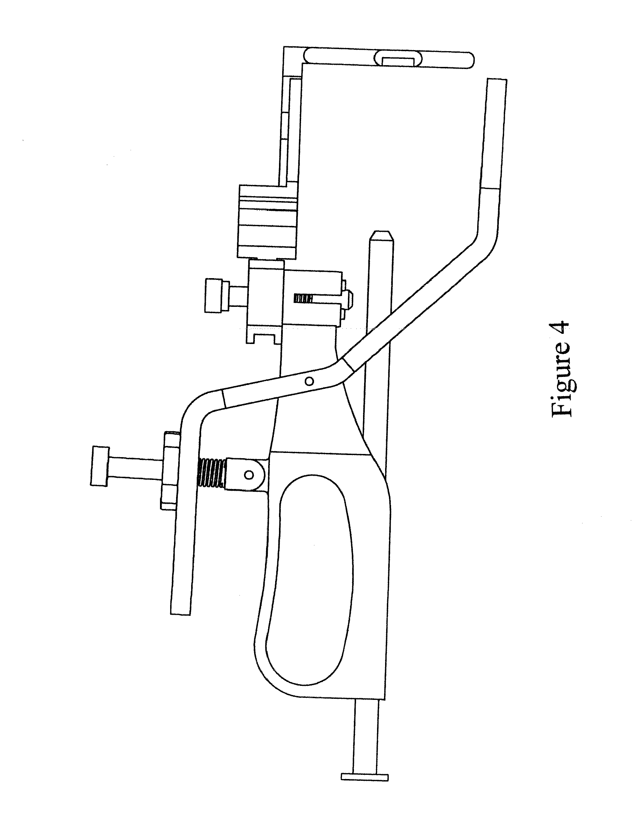

- Figure 4: is a side view of the alignment guide of Figure 2;
- Figure 5: is a perspective view from beneath of the alignment guide including the antiversion indicator means;
- Figure 6: is a perspective view from beneath of the alignment guide including alternative anteversion indicator means;
- Figure 7: is a detailed view of one arrangement of the antiversion indicator means;
- Figure 8: is a detailed view of a second arrangement of the antiversion indicator means;
- Figure 9: is a side view of the alignment guide in position on the femoral head with the anteversion indicator means detached;
- Figure 10: is the arrangement of Figure 9 with the antiversion indicator means in position;
- Figure 11: is a view from beneath of the arrangement of Figure 10;
- Figure 12: is a close up view from the side of the arrangement of Figure 10 with the goniometer in position;
- Figure 13: is a side view of the arrangement of Figure 12 illustrating the angle of the location of the goniometer in relation to the knee; and

- Figure 14: is a perspective view of the arrangement of Figure 13 looking along the femur.

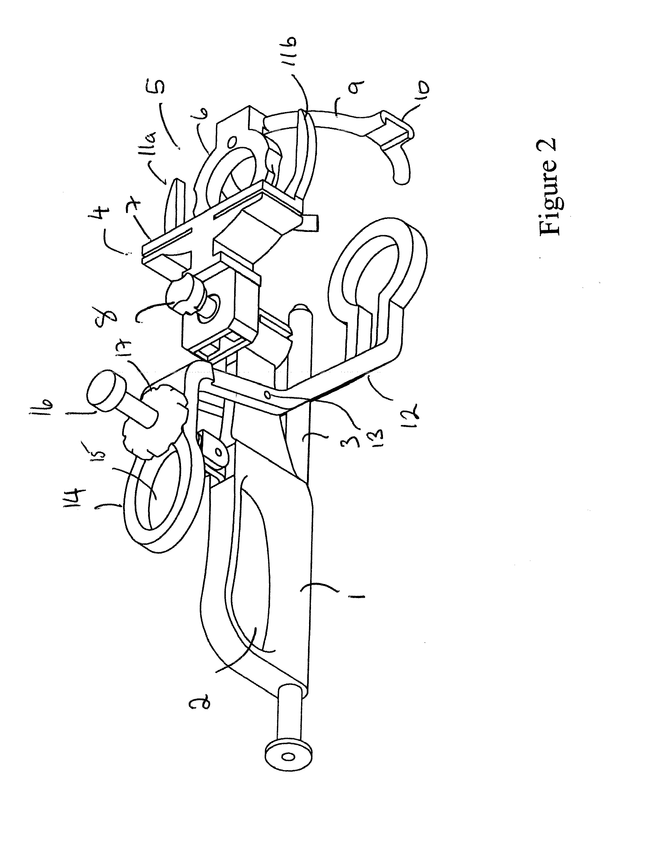

As illustrated in Figure 2, the alignment guide of the present invention comprises a support member 1 having a handle 2 through which in use the surgeon can place his fingers. A cannulated rod 3 passes through the support member. A locator arm 4 extends from the support member. The locator arm 4 has a locator member 5 located at its distal end. The locator member 5 comprises an annular component 6 which extends from a saw guide 7. The locator member is demountable from the support arm 4 via a locking means 8. Any suitable locking means may be used.

A notch guard 9 in the form of an at least partial ring extends from the locator member 6 and at right angles thereto. The notch guard 9 includes interlocking means 10 for connecting the notch guard with the antiversion indicator means.

Two indicator fingers 11a and 11b are included at the distal end of the locator arm 4. In the illustrated arrangement they extend from the saw guide 7. The locator fingers are shaped to provide, in use, an indication of the profile of the replacement femoral head.

A support arm 12 is connected to the support member 1 via a pivot 13. A support arm handle 14 is provided with an aperture 15 through which the surgeon can place his thumb. The support arm may be caused to move and subsequently locked in place by means of the screw 16 and the knurled knob 17

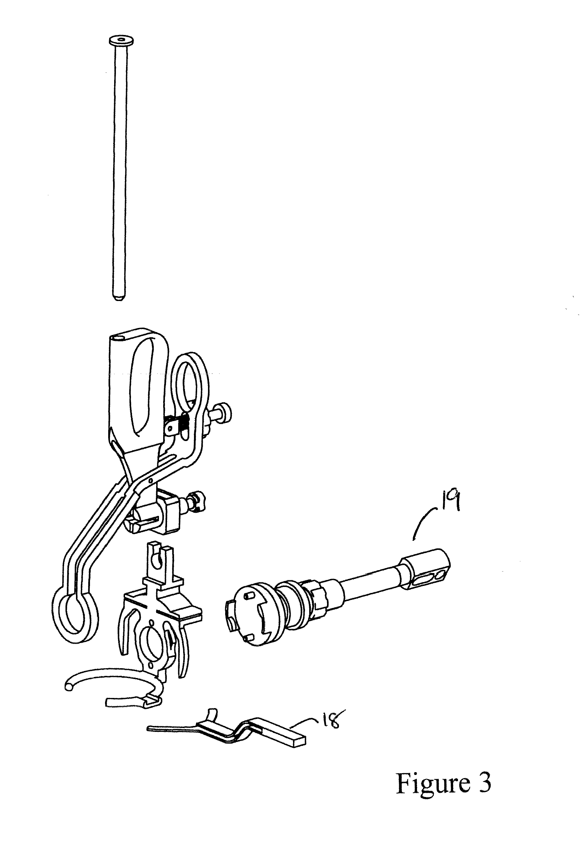

The separate components of the present invention including the antiversion indicator means 18 and the goniometer 19 are illustrated in Figure 3 which also illustrates one arrangement for the interlocking arrangement used in the illustrated embodiment for connecting the locating member to the locator arm.

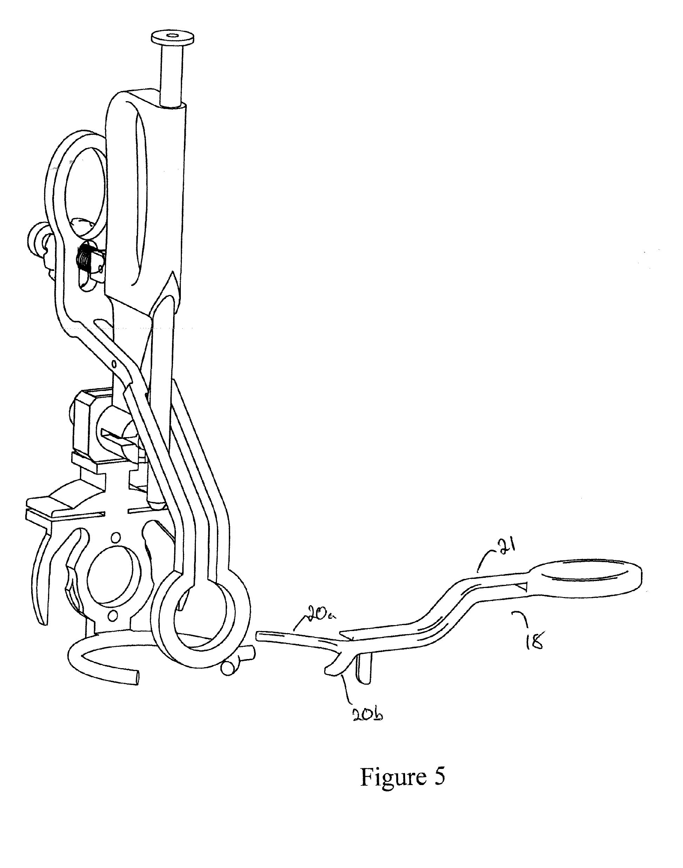

One arrangement for the antiversion indicator means 18 is illustrated in Figure 5. In this arrangement, the indicator is a fork arrangement having two tines 20a and 20b which when the antiversion indicator means is connected with the notch guard lie along respective portions of the ring of the notch guard 9. Interlocking means are provided on the antiversion indicator means to interlock with the corresponding arrangement on the notch guard.

It will be understood that the interlocking means on the notch guard will be provided in a position which will ensure that when the anteversion indicator means is placed in position, the tail 21 will lie along the desired angle.

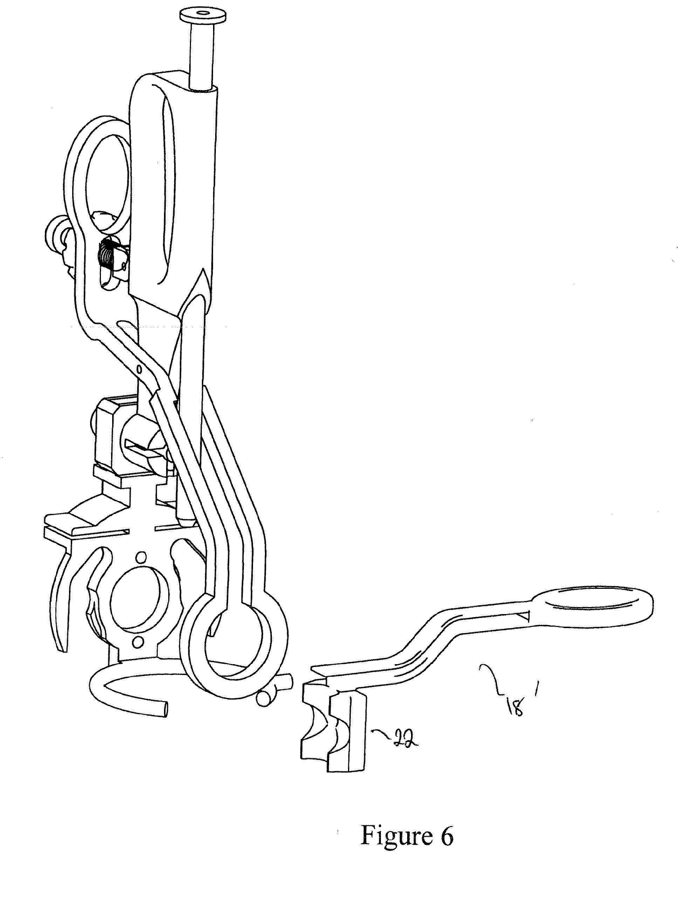

An alternative anteversion indicator means 18' is illustrated in Figure 6. In this arrangement a biting element 22 is provided comprising a toothed block.

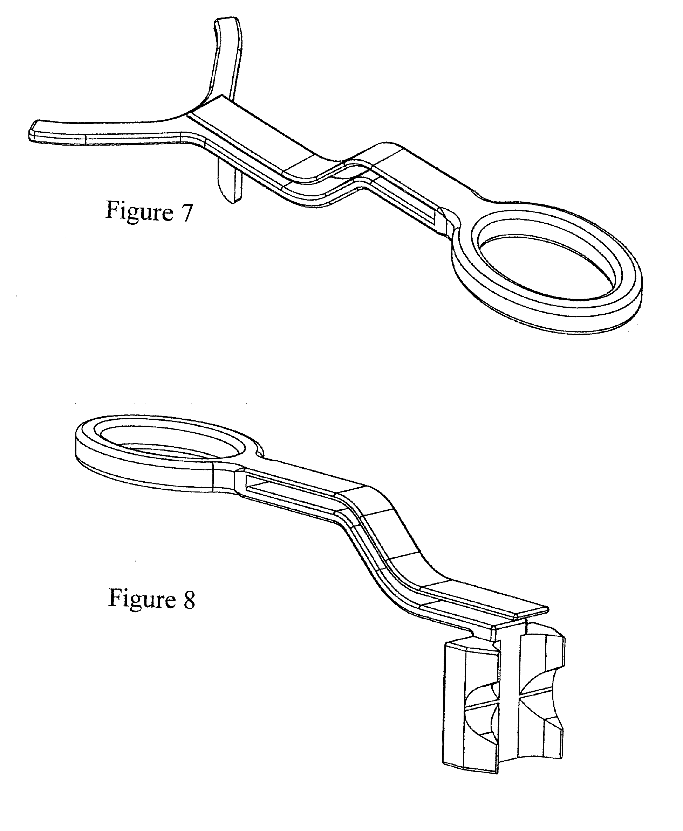

The anteversion indicator means are illustrated in more detail in Figures 7 and 8.

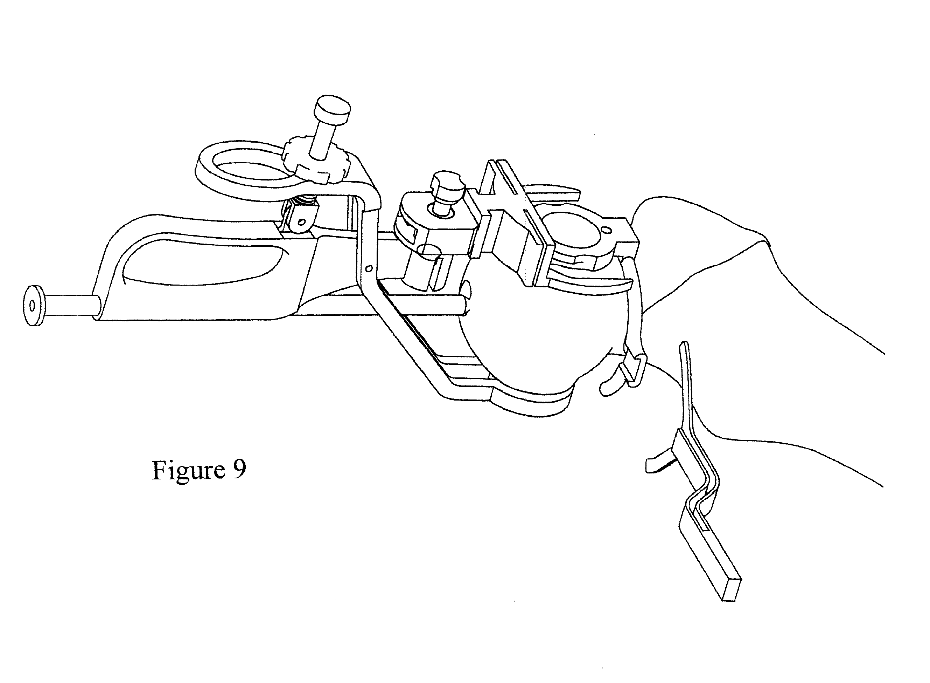

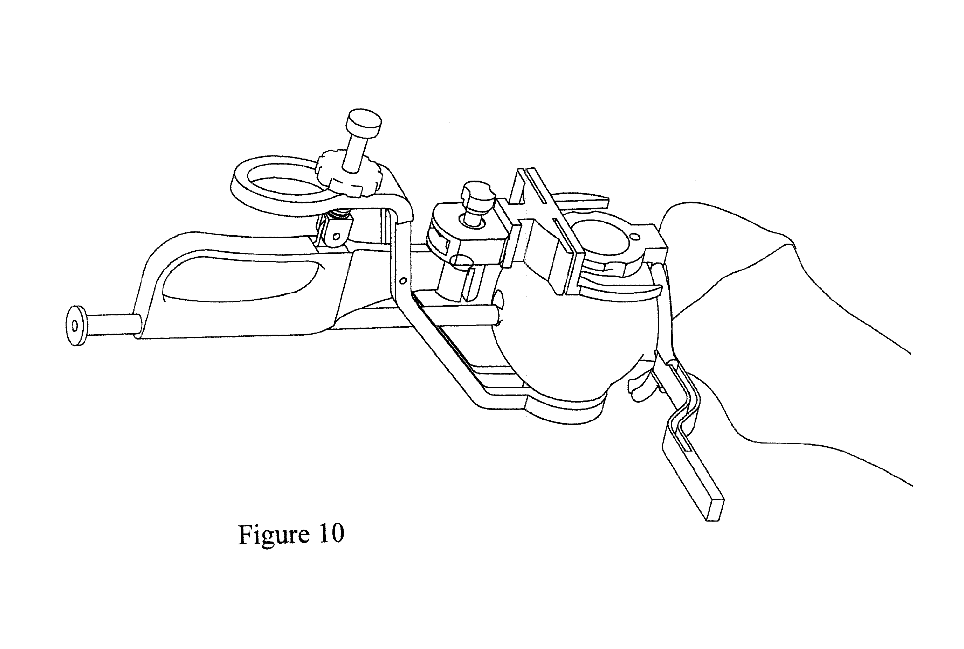

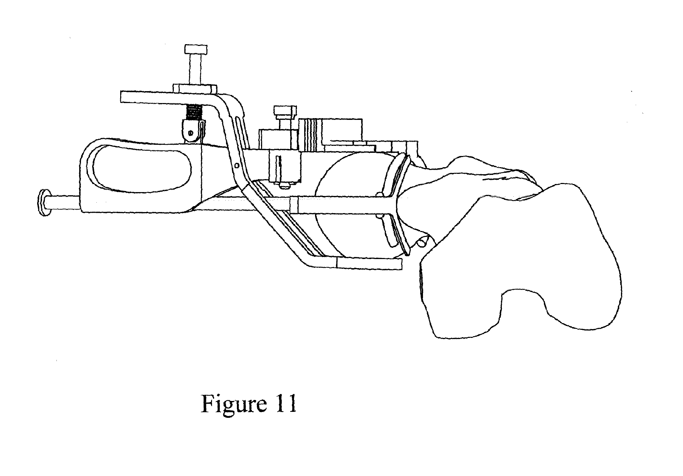

In use, once the surgeon has assessed the size of the femoral head, preferably using calipers, and the appropriate sized locator means has been selected, the alignment guide of the present invention is placed around the femoral head as illustrated in Figure 9. The anteversion indicator means is then connected to the alignment guide as illustrated in Figure 10. The positioning of the alignment guide can then be adjusted until the tail of the anteversion indicator means lines up with the appropriate part of the neck as illustrated in Figure 11.

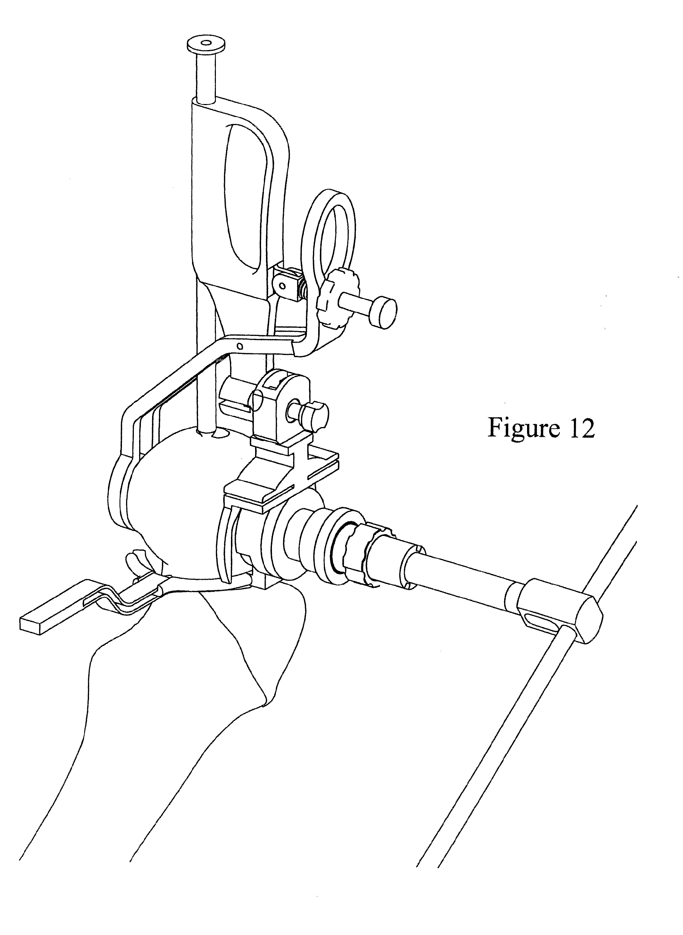

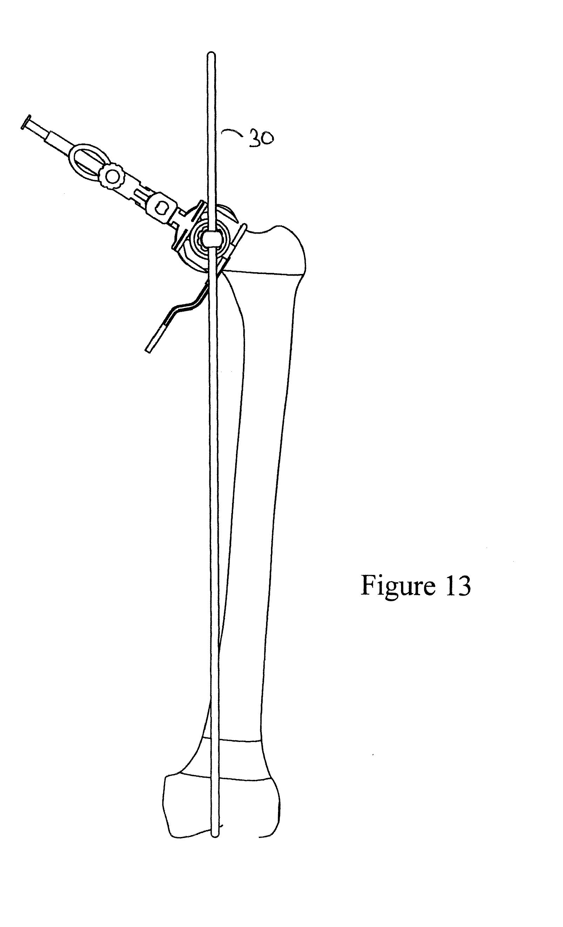

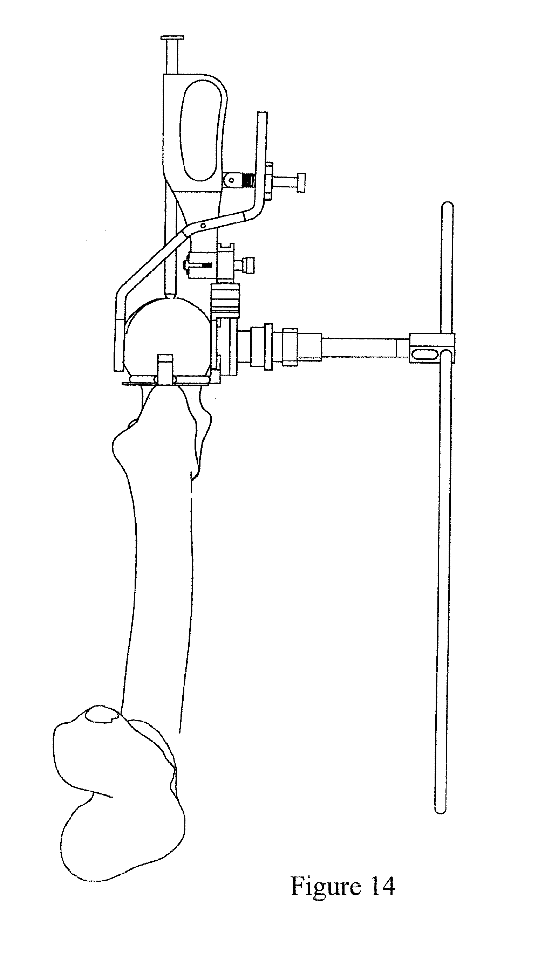

A goniometer may be connected to the locator means as indicated in Figure 12. The rod 30 of the goniometer should be pointed at the knee as illustrated in Figures 13 and 14. Again adjustments of the alignment guide can be undertaken until the orientation has been optimised.

## Claims

1. An alignment guide for use in femoral head surgery comprising:
a support member;
a cannulated rod supported by, and adjustable with respect to, the support member; and
a locator arm having a proximal end connected to the support member and a distal end having location means for location on a high point of the femoral head and a notch guard which in use will extend around at least a part of the femoral neck.

2. An alignment guide according to Claim 1 wherein the location means is of an annular configuration.

3. An alignment guide according to Claim 1 or 2 wherein the notch guard is a ring extending from the location means and generally angled thereto.

4. An alignment guide according to Claim 3 wherein the ring is a C-ring.

5. An alignment guide according to any one of Claims 1 to 4 wherein the alignment guide includes a support arm extending from the support member and connected thereto.

6. An alignment guide according to Claim 5 wherein the support arm includes contact means.

7. An alignment guide according to Claim 5 or 6 wherein at least one of the locator arm and the support arm is moveable from a first open position to a second clamping position.

8. An alignment guide according to Claim 7 wherein means for causing the movement is included.

9. An alignment guide according to any one of Claims 1 to 8 wherein the locator means includes two indicator fingers.

10. An alignment guide according to any one of Claims 1 to 9 wherein the locator means is demountable.

11. An alignment guide according to any one of Claims 1 to 10 wherein the locator means includes a cutting guide.

12. An alignment guide according to any one of Claims 1 to 11 additionally including a goniometer.

13. An alignment guide according to any one of Claims 1 to 12 additionally including anteversion indicating means.

14. An alignment guide according to Claim 13 wherein the anteversion indicating means is plugged onto the notch guard.

15. An alignment guide according to Claim 13 or 14 wherein the anteversion indicating means is a Y shaped fork.

16. An alignment guide according to Claim 13 or 14 wherein the anteversion indicating means includes a biting element.

17. An alignment guide according to Claim 16 wherein the biting element is a block having a concave face having teeth extending therefrom.

18. A kit comprising at least one alignment guide according to any one of Claims 1 to 17, a goniometer and an anteversion alignment guide.

19. A kit according to Claim 18 wherein the kit includes a plurality of locator means representing various sizes of resurfacing heads.
